# EUROPEAN PATENT SPECIFICATION

(11) **EP 3 094 246 B1**
(45) Date of publication and mention of the grant of the patent: **06.03.2024**
(21) Application number: 15700296.5
(22) Date of filing: 13.01.2015
(51) Int. Cl.: A61B 5/053, A61B 5/1455, A61B 5/01, A61B 5/145, A61B 5/07, A61B 5/00, A61B 5/0537, A61B 5/0538, A61B 10/00

(54) **IMPLANTABLE SENSOR**
IMPLANTIERBARER SENSOR
CAPTEUR IMPLANTABLE

(30) Priority: 16.01.2014 US 201414157298
(43) Date of publication of application: 23.11.2016
(73) Proprietor: D.T.R. Dermal Therapy Research Inc., London, ON N5X 2N9 (CA)
(72) Inventor: RUSU, Ana, S-192 51 Sollentuna (SE); DUENAS, Saul Alejandro Rodriguez, S-176 68 Järfälla (SE); OLLMAR, Stig, S-141 46 Huddinge (SE)
(74) Representative: Groth & Co. KB
(86) International application number: PCT/EP2015/050484
(87) International publication number: WO 2015/107040

(56) References cited:
- WO-A1-2009/121392
- US-A- 5 970 986
- US-A1- 2006 036 286
- US-A1- 2007 010 759
- US-B1- 6 198 965
- Muhammad Waqar Hussain ET AL: "Low Power Implantable ASIC for Bio-Impedance Measurements A Thesis submitted for the degree of Master of Science in System on Chip Design Supervised By", , 1 August 2012 (2012-08-01), XP55169435, Retrieved from the Internet: URL:http://www.diva-portal.org/smash/get/d iva2:570016/FULLTEXT01.pdf [retrieved on 2015-02-12]

## Description

### FIELD OF THE INVENTION

The present invention relates to the field of implantable medical devices and implantable sensor for measuring bio-impedance. In particular embodiments of the present invention, it relates to sensors that can be implanted into a body to detect or measure at least one physiological parameter of the body such a blood glucose levels.

### BACKGROUND OF THE INVENTION

Up to the present time, effective monitoring and follow-up of user related conditions or parameters such as different physiological parameters, health status, drug compliance has been limited to user's wearing implantable pacemakers and implantable cardioverters-defibrillators (ICDs). Current devices allow access to multiple critical data points reflecting device functionality and overall clinical condition of the user. The most recent advancements in device follow-up has provided for easier access to device stored data by utilizing wireless connectivity and internet based access to data as complement to information derived in point of care settings.

Nevertheless, despite these improvements in technology, there is a need of an improved system for effective monitoring and follow-up of user related conditions or parameters such as different physiological parameters including hydration, glucose levels etc., health status, drug compliance, in connection with organ transplantations to monitor the vitality of an organ during transportation from donor to recipient, and to monitor signs or rejection, infections or ischemia, monitor the ovarian cycle using e.g. temperature, and monitoring glucose and hydration to identify alertness of aviators, truck drivers etc. There is clearly a need of such a system that can be used with implantable sensors that are small, reliable, easy and cheap to produce and that can be carried over extended periods of time without any need for re-charge or change of battery. Obviously, implantable pacemakers and implantable cardioverters-defibrillators (ICDs) are not suitable for such a system.

In addition, it would be very beneficial to include an implantable sensor in such improved system. Implantable sensors are sensors configured to be implanted within living tissue, e. g. within a living patient. The patient may comprise an animal or a human. Such implantable sensors are typically used to monitor one or more physiological parameters associated with the patient. For example, an implantable sensor may monitor a patient' s blood or other body fluids for the presence or absence of a specific substance. Other implantable sensors may monitor the patient's body temperature. In general, implantable sensors may be used to provide valuable data that assists in diagnosing or treating an illness, or to help maintain or sustain a given level of physiological, chemical, or other activity or inactivity.

An area of high importance in which an implantable sensor and a monitoring system would be of great use is glucose monitoring or diabetes monitoring. At the present time, patients with diabetes rely on monitoring of blood glucose using an invasive blood glucose meter several times every day. Often this method involves drawing a small sample of blood, which is then tested directly for glucose level. There are numerous drawbacks to this method, for example, the patient have to draw samples of blood every day, several times a day at regular intervals, and there is some discomfort associated with drawing blood samples repeatedly. In addition, there is a margin of error, for example, the patient may forget to take a blood sample.

Present glucose sensors, which are typically used with some type of insulin-delivery system in order to treat diabetics, provide data needed to maintain the concentration of glucose within the patient at an acceptable level. Such glucose sensors must perform properly; otherwise, false data may be provided. Such false data (if acted upon) could result in the administration of an inappropriate amount of insulin, leading to death or serious injury. There is thus a critical need in the art for a sensor which is reliable and which can be monitored for proper function on a regular basis. Likewise, there is a need for a glucose sensor which must work properly within certain specific limits of accuracy.

Many implantable sensors require a power source, such as a battery, to power the sensor and transmitter and are therefore useful for only a limited period of time after implantation. After the on-board power source is depleted, an invasive operation, in addition to the initial implantation, will have to be made, if the device is to be removed or replaced.

Hence, there is also a need for an implantable device that can sense or detect one or more physiologic parameter values, and that can be remotely accessed by, for example, a hand held reader to obtain sensed parameters values in a non-invasive manner. No on-board power sources should be used so that the device will never need to be removed from an implantation site in order to replace an electrical power source, and can therefore remain implanted for an indefinite period of time.

In "Wireless Glucose Monitoring Watch enabled by an Implantable Self-sustaining Glucose sensor system" by Rai P. and Varadan V., Progress in Biomedical Optica and Imaging, Proceedings of SPIE8548, 2012, a system including an implantable glucose sensor that can be powered with inductive coupling is described. The sensor can communicate with a watch and glucose data can be displayed on the watch. The sensor described in this article has however only a limited working life since it consumes itself during use.

In Gupta et al, US2007276201, a system for monitoring strain as an indicator of biological conditions, such as spinal fusion, glucose levels, spinal loading, and heart rate is disclosed. The system includes an inter-digitated capacitance sensor, and RF transmitter, and an associated antenna, all of which are microminiature or microscopic in size and can be implanted in a biological host such as a human or animal. An inductively coupled power supply is also employed to avoid the need for implantation of chemical batteries. Power is provided to the sensor and transmitter, and data is transmitted from the sensor, when an external receiving device, such as a handheld RF ID type receiver, is placed proximate the location of the implanted sensor, transmitter and inductively coupled power supply. The implanted sensor, transmitter and inductively coupled power supply can be left in place permanently or removed when desired.

In Yang et al, US2004180391, in vivo or in vitro monitoring of chemical and biochemical species (e.g., pH, or glucose levels) in the interstitial fluid of patients or in a sample of a fluid to be analyzed is provided by a probe (10, 70, 210, 270). For in vivo monitoring, the probe is readily inserted by a minimally invasive method. Optical or electrochemical sensing methods are employed to detect a physical or chemical change, such as pH, color, electrical potential, electric current, or the like, which is indicative of the concentration of the species or chemical property to be detected. Visual observation by the patient may be sufficient to monitor certain biochemicals (e.g., glucose) with this approach. A CAP membrane allows high enzyme loadings, and thus enables use of microminiature probes, and/or diagnosis of low levels of the analyte(s), with sufficient signal-to-noise ratio and low background current.

In "A hydrogel-based implantable micromachined transponder for wireless glucose measurement" by Lei M. et al., Diabetes technology & Therapeutics, Vol. 8, No. 1, 2006, a hydrogel-based implantable wireless glucose sensor is described. The basic structure is a passive micromachined resonator coupled to a stimuli-sensitive hydrogel, which is confined between a stiff nanoporous membrane and a thin glass diaphragm.

In "Die Impedanzmessung zur Beurteilung von Ischämieschäden der humanen Leber in der Vorbereitung zur Transplantation", Gersing E., Langenbecks Arch Chir (1993) 378: 233-238, "Impedance spectroscopy on living tissue for determination of the state of organs", Gersing E., Bioelectrochemistry and Bioenergetics (1998) 45: 145-149, "Quantitative analysis of impedance spectra of organs during ischemia", Gheorghiu M, Gersing E, Gheorghiu E, Annals of the New York Academy of Sciences (1999) 873: 65-71, and "Messung der elektrischen Impedanz von Organen - Apparative Ausrustung fur Forschung und klinische Anwendung", Gersing E., Biomedizinische Technik (1991) 36: 6-11, impedance measurements in organ were studied.

WO 2009/121392 A1 discloses a device that has an electrically conductive probe consisting of several electrodes for measuring electrical impedance of the skin tissue of a diabetic patient. The electrically conducting probe and sensing device are adapted to obtain electrical impedance of tissue and ion concentration in tissue simultaneously. A blood glucose determination unit determines glucose concentration based on measured electrical impedance and determined ion concentration.

To conclude, despite these numerous attempts within the art, there is still a need of an improved system for effective monitoring and follow-up of user related conditions or parameters such as different physiological parameters including hydration, glucose levels etc., health status, drug compliance, in connection with organ transplantations to monitor the vitality of an organ during transportation from donor to recipient, and to monitor signs of rejection, infections or ischemia, monitor the ovarian cycle using e.g. temperature, and monitoring glucose and hydration to identify alertness of aviators, truck drivers etc. Furthermore, there is still a need for an improved implantable sensor that is small, reliable, easy and cheap to produce and that can be carried over extended periods of time without need for re-charge or change of battery.

### SUMMARY OF THE INVENTION

Disclosed herein is an implantable sensor for measuring or detecting one or more user related parameters, for example, physiologic parameters. The measured parameter can be remotely accessed by, for example, a hand held reader to obtain sensed parameters values in a non-invasive manner. The sensor does not use any on-board power sources and thus the sensor will never need to be removed from an implantation site in order to replace an electrical power source, and can therefore remain implanted for an indefinite period of time. Accordingly, the present invention provides for an effective monitoring and follow-up of user related conditions or parameters such as different physiological parameters including hydration, glucose levels etc., health status, drug compliance, in connection with organ transplantations to monitor the vitality of an organ during transportation from donor to recipient, and to monitor signs of rejection, infections or ischemia, monitor the ovarian cycle using e.g. temperature, and monitoring glucose and hydration to identify alertness of aviators, truck drivers etc. The present invention provides further an improved implantable sensor that is small, reliable, easy and cheap to produce and that can be carried over extended periods of time without need for re-charge or change of battery.

According to an aspect of the present invention, there is provided a device for measuring impedance in a subject, the device being configured to be implanted within the body of the subject and being configured to measure impedance within a body tissue of the subject resulting from an electrical current flowing through the body tissue, wherein the body tissue is sub-dermal or subcutaneous tissue of the subject. The device comprises one pair of injection electrodes configured for injection of electrical current into the body tissue, wherein the electrical current is passed from one of the injection electrodes to the other of the injection electrodes through the body and one pair of sensing electrodes configured to detect the resulting voltage caused by the current flowing between the pair of injection electrodes and through the body tissue. The device further comprises adetector operatively connected to the sensing electrodes and configured to receive the voltage detected by the sensing electrodes, wherein the detector is configured to measure the impedance of the body tissue based on the voltage detected by the pair of sensing electrodes. A microcontroller is operatively connected to the detector and being configured to receive impedance signals from the detector and to provide control signals to the current signal output circuit. Further, the device comprises a current signal output circuit operatively connected to the microcontroller and the injection electrodes and being configured to provide electrical current at predetermined frequencies to the injection electrodes and a powering and communication circuit including a coil is configured to be powered by an electromagnetic field produced by an external coil, the powering circuit being operatively connected to the microcontroller and configured to power the microcontroller, the current signal output circuit and the detector. The microcontroller is programmed to determine a glucose level in the subject by correlating the measured impedance with a predetermined relationship between impedance and blood glucose levels. The device for measuring impedance in a subject is a 2kHz to 2MHz bio-impedance sensor application-specific integrated circuit (ASIC), which is designed in 150nm CMOS technology and consumers 165µA at 1.8V when powered by an external reader.

A remote reader module can be used to energize the device, such as with electromagnetic energy, to thereby cause the device to sense the physiologic parameter values and to transmit the data representative thereof to the remote reader.

Due to its small size and the absence of a need of an on-board electrical power source, the sensor according to the present invention is particularly suitable for human implantation and can remain implanted for an indefinite period of time.

The detector in the implantable sensor may use one path to extract the I and Q components of the signal. The result of the I/Q demodulation is a DC signal, which entails that the extraction of the I and Q components can be performed when required or desired. This is in contrast to prior art I/Q demodulation in communication systems, where phase and amplitude change over time and the processing therefore has to be performed in parallel. The solution according to the present invention leads to significant reduction in power consumption since only one path needs to be active. This is of importance in the present invention since limited power can be extracted from the inductive coupling. This also entails that sensor itself can be made smaller.

According to embodiments of the present invention, the microcontroller is configured to communicate the measured impedance to an external device via the powering and communication circuit and wherein the monitoring engine is arranged in the external device.

According to embodiments of the present invention, the microcontroller is configured to communicate the measured impedance to an external device via the powering and communication circuit and wherein the monitoring engine is arranged in the external device and is configured to determine a glucose level in the subject by correlating the measured impedance with a predetermined relationship between impedance and blood glucose levels.

According to embodiments of the present invention, the current signal output circuit is configured to provide the injected current at a plurality of frequencies in a range between1 kHz to 3 MHz, and preferably within a range between 1.5 kHz and 2.5 MHz, and more preferably in a range between 1.90 kHz and 2 MHz.

According to embodiments of the present invention, a frequency generation circuit operatively connected to the detector and being configured to generate reference signals having a frequency between 5 kHz to 50 MHz, and preferably in a range between 10 kHz to 20 MHz and more preferably in a range between 16 kHz to 16 MHz, and to deliver the reference signals to the detector.

According to embodiments of the present invention, the I/Q demodulator comprises a multiplier configured to multiply the received voltage with the reference signal.

According to embodiments of the present invention, the detector comprises a voltage amplifier for amplifying the voltage sensed by the sensing electrodes.

According to embodiments of the present invention, the detector further comprises a low pass filter for filtering the amplified signals.

According to embodiments of the present invention, the device is configured to be implanted within the body of the subject sub-dermally or subcutaneously.

Disclosed herein is also a device for measuring impedance in a subject, the device being configured to be implanted within the body of the subject and being configured to measure impedance within a body tissue of the subject resulting from an electrical current flowing through the body tissue using a two-point technology, wherein the body tissue is sub-dermal or subcutaneous tissue of the subject, comprising: one pair of injection electrodes configured for injection of electrical current into the body tissue, wherein the electrical current is passed from one of the injection electrodes to the other of the injection electrodes through the body; one pair of sensing electrodes configured to detect the resulting voltage caused by the current flowing between the pair of injection electrodes and through the body tissue, wherein the injection electrodes and the sensing electrodes are the same electrodes. Furthermore, the device comprises a current signal output circuit operatively connected to the microcontroller and the injection electrodes and being configured to provide electrical current at predetermined frequencies to the injection electrodes, a detector operatively connected to the sensing electrodes and configured to receive the voltage detected by the sensing electrodes, wherein the detector is configured to measure the impedance of the body tissue based on the voltage detected by the pair of sensing electrodes and a microcontroller operatively connected to the detector and being configured to receive impedance signals from the detector and to provide control signals to the current signal output circuit. A powering and communication circuit including a coil configured to be powered by an electromagnetic field produced by an external coil, the powering circuit being operatively connected to the microcontroller and configured to power the microcontroller, the current signal output circuit and the detector.

Further disclosed herein is a method for measuring impedance in a subject using a device being configured to be implanted within the body of the subject and being configured to measure impedance within a body tissue of the subject resulting from an electrical current flowing through the body tissue, wherein the body tissue is sub-dermal or subcutaneous tissue of the subject. The method comprises on a general level the following steps:
providing power for the impedance measurement by receiving power at a coil via an electromagnetic field produced by an external coil;
providing electrical current at predetermined frequencies to the injection electrodes;
injecting electrical current into the body tissue via one pair of injection electrodes, wherein the electrical current is passed from one of the injection electrodes to the other of the injection electrodes through the body;
sensing or detecting the resulting voltage caused by the current flowing between the pair of injection electrodes and through the body tissue at one pair of sensing electrodes; and
measuring or determining the impedance of the body tissue based on the voltage detected by the pair of sensing electrodes.

Disclosed herein is further an I/Q (In-phase/Quadrature) demodulation is performed in the step of measuring on one signal path for extraction of the I and Q components, respectively, wherein a sensed voltage is received from the sensing electrodes as input and an output of the I/Q demodulation is at least one DC signal.

The method may further comprise determining or monitoring at least one physiological parameter of the body of the subject by correlating the measured impedance with a predetermined relationship between impedance and at least one physiological parameter.

The method may further comprise the step of monitoring at least one physiological parameter comprises determining a glucose level in the subject by correlating the measured impedance with a predetermined relationship between impedance and blood glucose levels.

The method further comprises communicating the measured impedance and/or a determined value of the physiological parameter (such as a glucose level) to an external device via the coil using electromagnetic fields. If the measured impedance is communicated to the external device, the determination of the physiological parameter can be performed in the external device and the step of communicating is executed before the step of determining at least one physiological parameter.

The method may further comprise the step of providing electrical current at predetermined frequencies to the injection electrodes comprises providing current for the injection electrodes at a plurality of frequencies in a range between1 kHz to 3 MHz, and preferably within a range between 1.5 kHz and 2.5 MHz, and more preferably in a range between 1.90 kHz and 2 MHz.

The method may further comprise generating reference signals having a frequency between 5 kHz to 50 MHz, preferably in a range between 10 kHz to 20 MHz and more preferably in a range between 16 kHz to 16 MHz for the I/Q demodulation.

Further advantageous embodiments of the device according to the present invention and further advantages with the present invention emerge from the dependent claims and the detailed description of embodiments.

As understood, there are a number of further application in which the present invention can be used.

For example, by measuring vaginal impedance of a woman, the fertility cycle could be monitored and a fertility status may be determined. It has been shown by Bartos L., "Vaginal impedance measurements used for mating in the rat", Laboratory Animals 1977; 11: 53-56 and in Bartos L, Sedlacek J., "Vaginal impedance measurements used for mating in the guinea-pig", Laboratory Animals 1977; 11: 57-58, that the vaginal impedance of rats discloses a sharp peak (or drop) at time of ovulation.

The monitoring engine may be configured to monitor the fertility cycle and determine a fertility status. For example, a sharp peak (or drop) in the vaginal impedance may indicate time of ovulation.

Moreover, glucose management or monitoring is also of high importance for athletes. The present invention may be very useful for athletes to monitor their glucose levels during, for example, exercise and competition.

Yet another application is to monitor hydration and glucose levels, for example, to detect or monitor diabetic hyperosmolar syndrome, which is a serious condition that develops when blood sugar reaches a very high level. At this level, the blood becomes thick and syrupy, causing diabetic hyperosmolar syndrome. Excess sugar passes from your blood into your urine, triggering a filtering process that draws tremendous amounts of fluid from your body. Diabetic hyperosmolar syndrome usually affects people with type 2 diabetes, and may develop in people who haven't yet been diagnosed with diabetes. Left untreated, diabetic hyperosmolar syndrome can lead to life-threatening dehydration. Prompt medical care is essential.

In addition to monitoring of organs in the context of transplantation, from harvesting the organ from the donor to its implantation in the recipient, the present device could also be used to monitor the growth process of artificial organs, where the implanted sensor could be part of the matrix on which the artificial organ is grown, and stay as an integrated part of the full grown organ after implantation.

According to a further aspect disclosed herein, there is provided a device for measuring impedance in an object, the device being configured to be implanted within the object or attached to the object and being configured to measure impedance of the object resulting from an electrical current flowing through the body tissue, comprising one pair of injection electrodes configured for injection of electrical current into the object, wherein the electrical current is passed from one of the injection electrodes to the other of the injection electrodes through the object and one pair of sensing electrodes configured to detect the resulting voltage caused by the current flowing between the pair of injection electrodes and through the object. A current signal output circuit is operatively connected to the microcontroller and the injection electrodes and being configured to provide electrical current at predetermined frequencies to the injection electrodes and a detector operatively connected to the sensing electrodes and configured to receive the voltage detected by the sensing electrodes, wherein the detector is configured to measure the impedance of the object based on the voltage detected by the pair of sensing electrodes. A microcontroller operatively connected to the detector and being configured to receive impedance signals from the detector and to provide control signals to the current signal output circuit; and a powering and communication circuit including a coil configured to be powered by an electromagnetic field produced by an external coil, the powering circuit being operatively connected to the microcontroller and configured to power the microcontroller, the current signal output circuit and the detector. In embodiment of the present invention, the object is an organ intended for transplantation, or a section of the female reproductory tract.

Disclosed herein is also an optical detecting unit including LED's and a detector is arranged in the implantable sensor. The LED's may be two LED with different wavelengths to monitor oxygenated and deoxygenated blood that has different optical spectra and the detector may then be used to monitor oxygen saturation level. A number of other tissue conditions and analytes could be detected at the same time by choosing at least two LEDs of specific wavelengths, e.g. kreatinine which is a substance reflecting reduced kidney function. Other analytes reflect reduced liver function, and also general indicators such as temperature (thermistor), potassium level, sodium level, and pH could be included in the "button sized" sensor element and implanted for life. During transportation of organs for implantation, ischemia is the major concern, which could be detected both by EIS and optical spectral analysis, however after implantation rejection and infection become of interest, and it is important to decide what problem is at hand since the counter measures are different. Thus, by adding LEDs and optical detector to the impedance sensor, it would become more accurate in differential diagnosis.

Furthermore, edema such as pulmonary edema in patients suffering from heart diseases or pulmonary edema or cerebral edema in mountaineers during expeditions at high altitudes in order to monitor high altitude sickness or edema in divers to monitor divers sickness.

### BRIEF DESCRIPTION OF THE DRAWINGS

The present disclosure will now be described, for exemplary purposes, in more detail by way of embodiments and with reference to the enclosed drawings, in which:
Fig. 1 is a schematic view of a system for measuring impedance in a subject;
Fig. 2 is a schematic view of a computing device suitable for use in the system;
Fig. 3 is a schematic view of another computing device suitable for use in the system;
Fig. 4 is a schematic view of the computing device;
Fig. 5 is a schematic view of a reader module
Fig. 6 is a schematic view of the implantable impedance sensor;

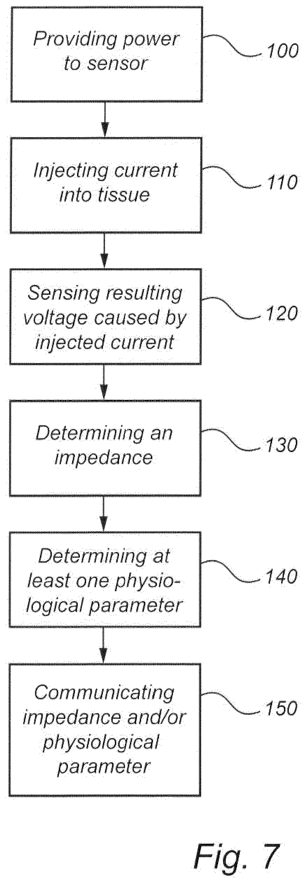

Fig. 7 is a schematic flow diagram **of** the method for determining an impedance and at least one physiological parameter; and
Fig. 8 is a schematic view of a further implantable impedance sensor.
Fig. 9 is a diagram showing measured impedance of sheep's liver and kidney using an example sensor, method and system
Fig. 10 is a diagram showing measured phase of sheep's liver and kidney using an example sensor, method and system.
Fig. 11 is a schematic view of another implantable sensor.

### DETAILED DESCRIPTION OF EMBODIMENTS

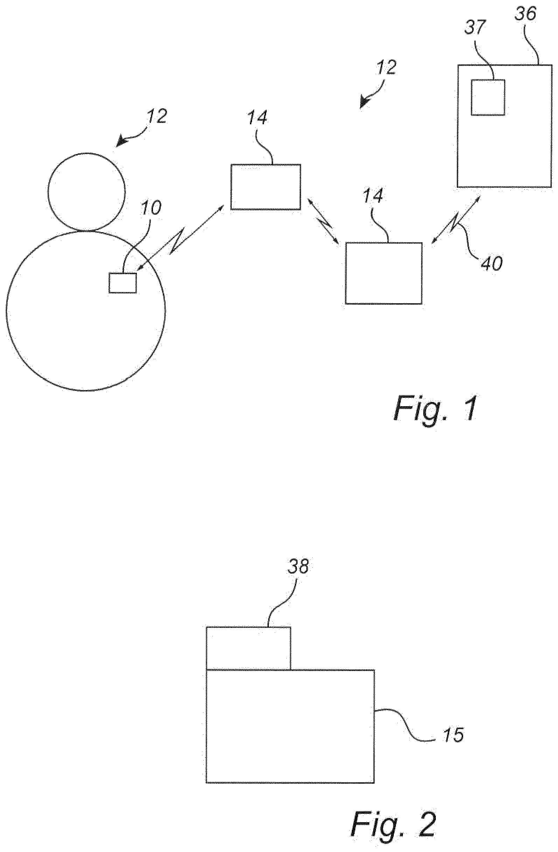

With reference first to Fig. 1, a system for measuring or monitoring user related conditions or parameters such as different physiological parameters including hydration, glucose levels etc., health status, drug compliance, in connection with organ transplantations to monitor the vitality of an organ during transportation from donor to recipient, and to monitor signs or rejection, infections or ischemia, monitor the ovarian cycle using e.g. temperature, and monitoring glucose and hydration to identify alertness of aviators, truck drivers etc. There is clearly a need of such a system that can be used with small, reliable, easy and cheap to produce and that can be carried over extended periods of time will be described. In preferred embodiments of the invention, the system uses a sensor that measures the impedance of body tissue and the impedance measurements are used to detect or monitor glucose levels.

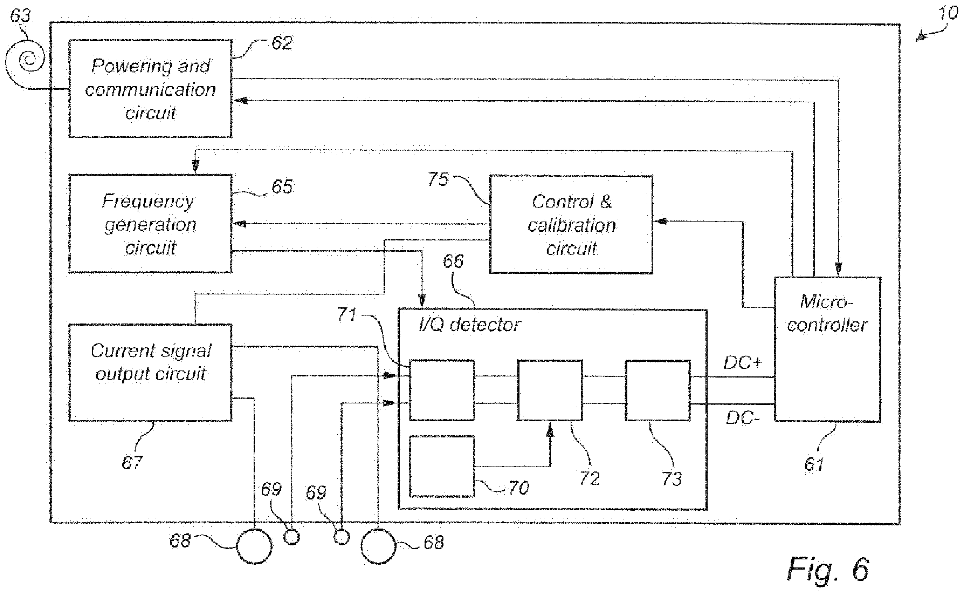

A sensor 10 for measuring electrical bio-impedance of a subject 12 is implanted into the subject, for example sub-dermally or sub-cutaneously. The implantable sensor 10 will be described in detail below with reference to Fig. 6 The sensor 10 is powered by an external reader module14 by using inductive coupling, for example, at frequencies around 10 - 15 MHz. The reader module 14 is capable of communicating with a microcontroller 61 of the sensor 10 (see e.g. fig. 6). For example, the reader module 14 may be arranged to perform half-duplex back-scattering serial communication with the sensor 10, also known as impedance modulation or load modulation. This technique works by reflecting electromagnetic waves back to the source. The short distance relative to the wavelength means that the reflected wave is received almost instantly. Therefore instead of receiving a pulse back the mutual inductance behaves as a feedback loop and changes the apparent impedance of the inductor. The change in inductance will then change the current that passes through the coil. The changed current will then change the amplitude of the voltage over the coil, and the data can be treated as an amplitude modulated signal. In principle any method that changes the impedance in the secondary resonator can be used to transmit data. For example, amplitude modulation for the downlink (from the reader 14 to the implantable device or sensor 10) by changing the voltage that is available in the sensor 10. The uplink (from the implantable device 10 to the reader 14) uses load shift keying, where the quality factor of the load is changed according to the data being sent. The load is sensed by using a transformer (not shown), which senses the current that passes through the coil used to transmit power. An envelope detector (not shown) followed by a band pass filter (not shown) and comparator (not shown) is used to recover the data.

The reader module14 and the sensor 10 includes LRC resonant circuits configured for frequencies in a range between 10 - 15 MHz for power transmission and signal reception (at the reader 14). The reader module 14 is configured to communicate with a computing device 15, for example, using wireless communication including infrared, BLUETOOTH^{®} wireless technology, 802.11a7b/g/n, cellular or other radio frequency communication systems.

The reader module is included in the computing device as shown in Fig. 2. For example, a reader module 38 may be connected or coupled to the computing device at a USB port of the computing device 15. The reader module may alternatively be included into the computing device as module.

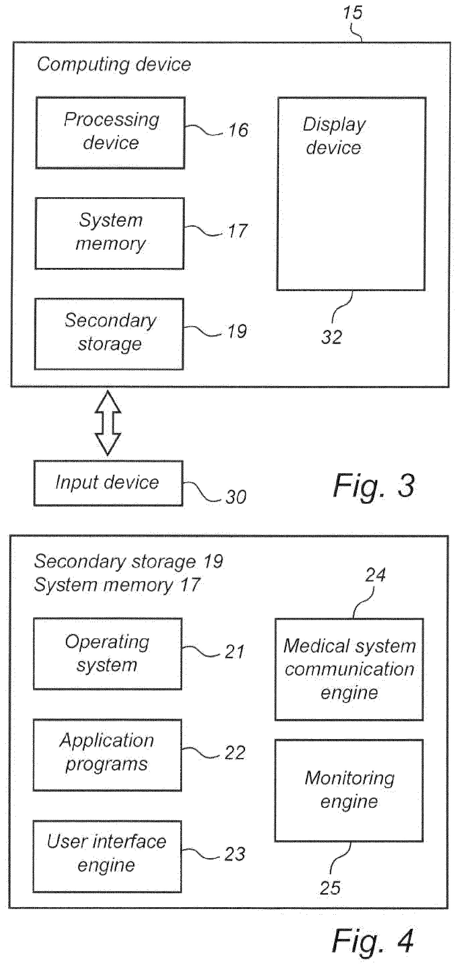

With reference to Fig. 3, the computing device 15 includes, at least one processing device 16, such as a central processing device (CPU). A variety of processing devices are available from a variety a manufacturers, for example, Intel or Advanced Micro Devices. The computing device also comprises a system memory 17.

Examples of computing devices suitable for use in the present system include, but without limitation to the mentioned examples, a desktop computer, a laptop computer, a tablet computer, a mobile computing device such as a smart phone (e.g. an iPhone^{®} or a phone based on Android OS), an iPod^{®}, an iPad^{®}, a mobile digital device or other mobile devices, or other devices configured to process digital instructions.

The system memory 17 includes read only memory and random access memory. A basic input/output system containing basic routines that act to transfer information within the computing device 15, such as start-up, is typically stored in the read only memory.

Further, the computing device 15 also includes a secondary storage 19 such as a hard disk drive, for storing digital data. The secondary storage 19 and associated computer readable media provide non-volatile storage of computer readable instructions (including programs and program modules), data structures and other data for the computing device 15.

Although the exemplary environment described herein employs a hard disk drive and a secondary storage, other types of computer readable storage media may be used. Examples of these other types of computer readable storage media include magnetic cassettes, flash memory cards, digital video disks, compact disc read only memories, digital versatile disk read memories, random access memories, or read only memories. Some embodiments include non-transitory media. Additionally, such computer readable storage media can include local storage or cloud-based storage.

As illustrated in Fig. 4, a number of program modules can be stored in the secondary storage 19 and/or system memory 17 including an operating system 21, one or more application programs 22, a user interface engine 23, a medical system communication engine 24 and a monitoring engine 25. The computing device 15 can utilize any suitable operating system, such as Microsoft Windows^{™},, Google Chrome^{™}, Apple OS, Android OS and any other operating systems suitable for a computing device. The monitoring engine may, be arranged to determine or monitor a physiological parameter such as a glucose level based on measured impedance. In Fig. 2 the computing device is capable of determining or monitoring a physiological parameter such as glucose based on impedance measurements. The impedance measurements are performed by the sensor 10 and the impedance data is then transmitted to the reader module 14 via a powering and communication module 62 of the sensor (see Fig. 6).

A user provides input to the computing device 15 through one or more input devices 30. Examples of input devices 30 include a keyboard, a mouse, a microphone, a touch sensor (such as a touchpad or touch sensitive display), an IR sensor or web-camera. The input device 30 is connected to the processing device 16 through an input/output interface that is coupled to a system bus (not shown).

The computing device 15 includes a display device 32 such as a monitor, liquid crystal display device, a projector or touch sensitive display device.

When used in a local area networking environment or a wide area networking environment (such as the Internet), the computing device 15 is typically connected to the network 40 (Fig. 1) through a network interface (not shown) such as an Ethernet interface. Other communication devices may be used. For example, the computing device 15 may include a modem for communicating across the network.

The computing device 15 is capable of communicating with, for example, a health care provide unit 36 via the network 40 using the medical system communication engine 24. The health care provider unit 36 comprises a patient portal 37, wherein an authorized user such as a medical doctor can access patient information via the patient portal 37. The computing device 15 uploads information, for example, related to measure physiological parameters of the subject or patient to the health care provide unit 36. An authorized user, e.g. a medical doctor, can access the uploaded information via the patient portal 37. Other information such health status, drug compliance, etc. can also be uploaded to the health care provide unit from the computing device 15. An authorized user may also communicate with the patient via the patient portal 37, for example, send a prescription of a drug or send updated information related to health status of the patient. Other user related conditions or parameters such as different physiological parameters including hydration, glucose levels etc., health status, drug compliance, in connection with organ transplantations to monitor the vitality of an organ during transportation from donor to recipient, and to monitor signs of rejection, infections or ischemia, monitor the ovarian cycle using e.g. temperature, and monitoring glucose and hydration to identify alertness of aviators, truck drivers etc. can also be monitored or followed up in the present system 8.

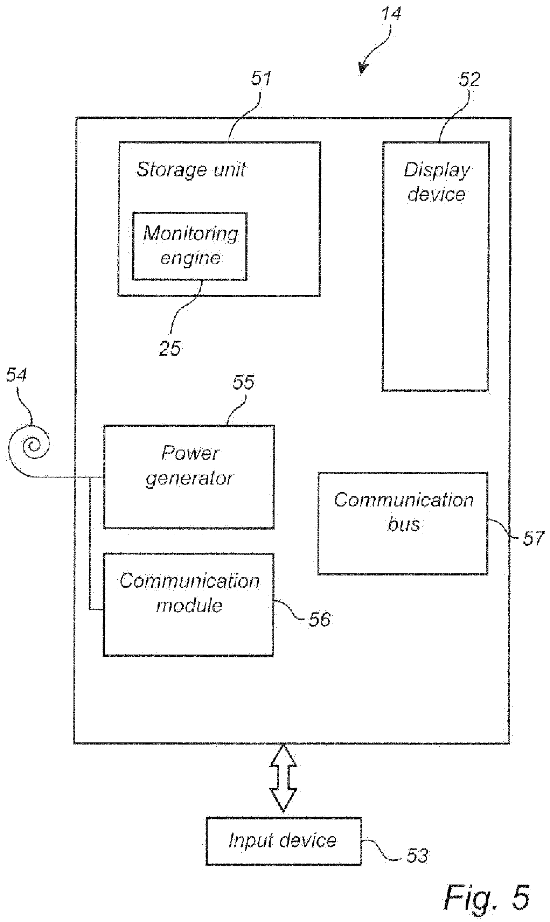

The monitoring engine 25 may be included in a storage unit 51 of the reader module 14 as illustrated in Fig. 5, e.g. a read only memory and random access memory and a secondary storage such as a hard disk drive, for storing digital data. The secondary storage and associated computer readable media provide non-volatile storage of computer readable instructions (including programs and program modules), data structures and other data for the reader device. Although the exemplary environment described herein employs a hard disk drive and a secondary storage, other types of computer readable storage media may be used. Examples of these other types of computer readable storage media include magnetic cassettes, flash memory cards, digital video disks, compact disc read only memories, digital versatile disk read memories, random access memories, or read only memories. Non-transitory media may be used. Additionally, such computer readable storage media can include local storage or cloud-based storage.

The reader module 14 may also include devices such as a display device 52 such as a monitor, liquid crystal display device, a projector or touch sensitive display device and an input device 53 such as a keyboard, a mouse, a microphone, a touch sensor (such as a touchpad or touch sensitive display), an IR sensor or web-camera.

The reader module 14 further comprises a coil 54 for producing electromagnetic fields for powering the sensor 10. The coil 54 is connected to power generator 55 configured to generate the current and voltage for the electromagnetic field and a communication module 56 for receiving transmitted data from the sensor 10.

The reader module 14 may also comprise a communication bus 57 for connection to the computing device 15, for example, via direct connection via a USB port (as shown in Fig. 5) or wirelessly, for example, via IR communication or via BLUETOOTH^{®}.

Turning now to Fig. 6, the implantable impedance device or impedance sensor will be discussed in more detail. Fig. 6 shows a block diagram of a sensor.

A powering and communication circuit 62 comprising analog circuits provides power to the sensor 10. The powering and communication circuit comprises a coil 63 for external powering by the reader module 14 using inductive coupling and the powering and communication circuit 62 is also configured to establish a communication mechanism with the reader module 14 using, for example, half duplex back-scattering serial technique. The powering and communication circuit 62 includes a full-wave rectifier circuit 64 which resonates with the coil 63, for example, at frequencies in a range between 10 - 15 MHz. The input to the powering and communication circuit 62 is an electromagnetic field produced by the coil 13 of the reading module 14. Output of the powering and communication circuit 62 is a DC voltage. The powering and communication circuit 62 is operatively connected to the microcontroller 61.

A frequency generation circuit 65 is configured to generate frequency reference clocks from signals having a frequency between 5 kHz to 50 MHz, and preferably in a range between 10 kHz to 20 MHz and more preferably in a range between 16 kHz to 16 MHz. These frequencies are used to generate sinusoidal current and I/Q waveforms for the I/Q impedance detection mechanism performed in an I/Q detector 66.

A current signal output circuit 67 is operatively connected to a pair of injection electrodes 68 and is configured to provide electrical current at predetermined frequencies to the injection electrodes 68. The injection electrodes 68 is configured to inject the electrical current into the body tissue, wherein the electrical current is passed from one of the injection electrodes to the other of the injection electrodes through the body. The current signal output circuit 67 is configured to provide the injected current at a plurality of frequencies in a range between1 kHz to 3 MHz, and preferably within a range between 1.5 kHz and 2.5 MHz, and more preferably in a range between 1.90 kHz and 2 The frequencies are 1.95 kHz, 3.9 kHz, 7.8125 kHz, 15.625 kHz, 31.25 kHz, 62.5 kHz, 125 kHz, 250 kHz, 500 kHz, 1MHZ and 2 MHz.

A pair of sensing electrodes 69 is configured to detect the resulting voltage caused by the current flowing between the pair of injection electrodes 68 and through the body tissue. The sensing electrodes 69 are operatively connected to the detector 66, which receives the sensed voltage. The detector 66 comprises circuit for generating sinusoidal current waveform 70, amplifying circuits 71 for amplifying sensed voltage, multiplier 72 for multiplying the voltage with I/Q reference signals and low pass filter circuit 73 for low pass filtering the signals.

The detector 66 has one path to extract the I- and Q-components of the signal. The result of the I/Q demodulation is a DC signal, which entails that the extraction of the I and Q components can be performed when required. This is in contrast to prior art I/Q demodulation in communication systems, where phase and amplitude change over time and the processing therefore has to be performed in parallel.

A control and calibration circuit 75 is operatively connected to the microcontroller 61, current frequency generation circuit 65, the current signal output circuit 67 and the detector 66. The control and calibration circuit 75 is configured to control and/or calibrate the different circuits and to communicate with the microcontroller 61.

According to the present disclosure not covered by the invention, there is provided a method for measuring impedance in a subject using a device being configured to be implanted within the body of the subject and being configured to measure impedance within a body tissue of the subject resulting from an electrical current flowing through the body tissue, wherein the body tissue is sub-dermal or subcutaneous tissue of the subject [organs for transplantation start outside the body]. The method comprises on a general level the following steps:
providing, 100, power for the impedance measurement by receiving power at a coil via an electromagnetic field produced by an external coil;
injecting, 110, electrical current into the body tissue via one pair of injection electrodes, wherein the electrical current is passed from one of the injection electrodes to the other of the injection electrodes through the body;
sensing, 120, the resulting voltage caused by the current flowing between the pair of injection electrodes and through the body tissue at one pair of sensing electrodes;
measuring or determining, 130, the impedance of the body tissue based on the voltage detected by the pair of sensing electrodes.

An I/Q (In-phase/Quadrature) demodulation is performed in the step of measuring 130 on one signal path for extraction of the I and Q components, respectively, wherein a sensed voltage is received from the sensing electrodes as input and an output of the I/Q demodulation is at least one DC signal.

The method further comprises determining or monitoring 140 at least one physiological parameter of the body of the subject by correlating the measured impedance with a predetermined relationship between impedance and at least one physiological parameter.

The step of monitoring 140 at least one physiological parameter comprises determining a glucose level in the subject by correlating the measured impedance with a predetermined relationship between impedance and blood glucose levels.

The method further comprises communicating 150 the measured impedance and/or a determined value of the physiological parameter (such as a glucose level) to an external device via the coil using electromagnetic fields. If the measured impedance is communicated to the external device, the determination of the physiological parameter can be performed in the external device and the step of communicating 150 is executed before the step of determining 140 at least one physiological parameter.

The at least one physiological parameter include body temperature, hydration levels, hormone levels, lactate levels, pH, pO2, other specific ions or molecules, local pressure inside brain or scull

The step of providing, 130, electrical current at predetermined frequencies to the injection electrodes comprises providing current for the injection electrodes at a plurality of frequencies in a range between1 kHz to 3 MHz, and preferably within a range between 1.5 kHz and 2.5 MHz, and more preferably in a range between 1.90 kHz and 2 MHz.

The method further comprises generating reference signals having a frequency between 5 kHz to 50 MHz, an preferably in a range between 10 kHz to 20 MHz and more preferably in a range between 16 kHz to 16 MHz for the I/Q demodulation.

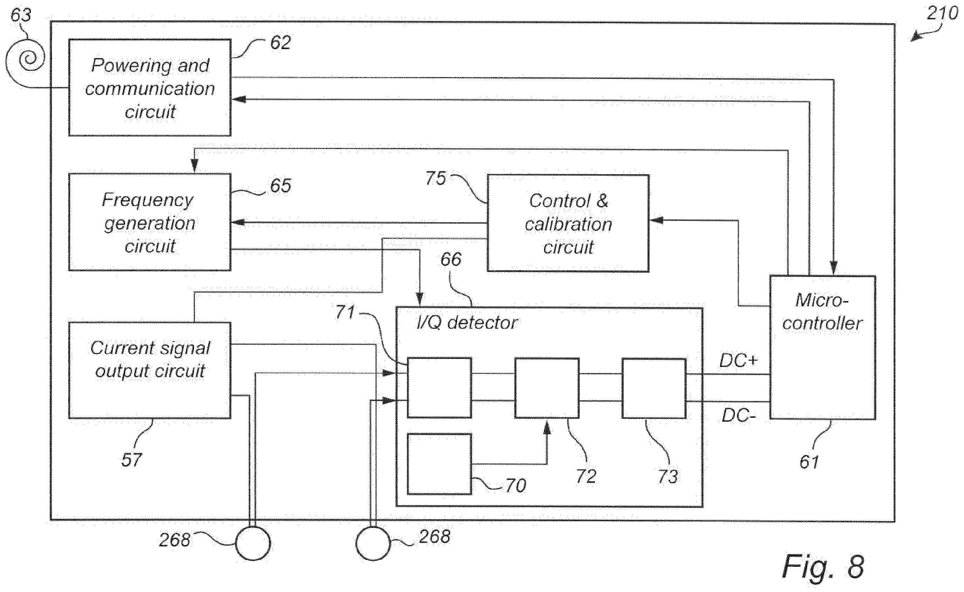

With reference now to Fig. 8, another implantable impedance device or impedance sensor will be discussed in more detail. Fig. 8 shows a block diagram of this sensor.

A powering and communication circuit 62 comprising analog circuits provides power to the sensor 210. The powering and communication circuit comprises a coil 63 for external powering by the reader module 14 using inductive coupling and the powering and communication circuit 62 is also configured to establish a communication mechanism with the reader module 14 using, for example, half duplex back-scattering serial technique. The powering and communication circuit 62 includes a full-wave rectifier circuit 64 which resonates with the coil 63, for example, at frequencies in a range between 10 - 15 MHz. The input to the powering and communication circuit 62 is an electromagnetic field produced by the coil 13 of the reading module 14. Output of the powering and communication circuit 62 is a DC voltage. The powering and communication circuit 62 is operatively connected to the microcontroller 61.

A frequency generation circuit 65 is configured to generate frequency reference clocks from signals having a frequency between 5 kHz to 50 MHz, and preferably in a range between 10 kHz to 20 MHz and more preferably in a range between 16 kHz to 16 MHz. These frequencies are used to generate sinusoidal current and I/Q waveforms for the I/Q impedance detection mechanism performed in an I/Q detector 66.

A current signal output circuit 67 is operatively connected to a pair of electrodes 268 and is configured to provide electrical current at predetermined frequencies to the electrodes 268. The electrodes 268 are configured to inject the electrical current into the body tissue, wherein the electrical current is passed from one of the electrodes 268 to the other of the electrodes 268 through the body. The current signal output circuit 67 is configured to provide the injected current at a plurality of frequencies in a range between1 kHz to 3 MHz, and preferably within a range between 1.5 kHz and 2.5 MHz, and more preferably in a range between 1.90 kHz and 2 MHz. The frequencies are 1.95 kHz, 3.9 kHz, 7.8125 kHz, 15.625 kHz, 31.25 kHz, 62.5 kHz, 125 kHz, 250 kHz, 500 kHz, 1MHZ and 2 MHz.

The resulting voltage caused by the current flowing between the pair of electrodes 268 and through the body tissue is detected at the electrodes 268. The electrodes 69 are also operatively connected to the detector 66, which receives the sensed voltage. The detector 66 comprises circuit for generating sinusoidal current waveform 70, amplifying circuits 71 for amplifying sensed voltage, multiplier 72 for multiplying the voltage with I/Q reference signals and low pass filter circuit 73 for low pass filtering the signals.

The detector 66 has one path to extract the I- and Q-components of the signal. The result of the I/Q demodulation is a DC signal, which entails that the extraction of the I and Q components can be performed when required. This is in contrast to prior art I/Q demodulation in communication systems, where phase and amplitude change over time and the processing therefore has to be performed in parallel.

A control and calibration circuit 75 is operatively connected to the microcontroller 61, current frequency generation circuit 65, the current signal output circuit 67 and the detector 66. The control and calibration circuit 75 is configured to control and/or calibrate the different circuits and to communicate with the microcontroller 61.

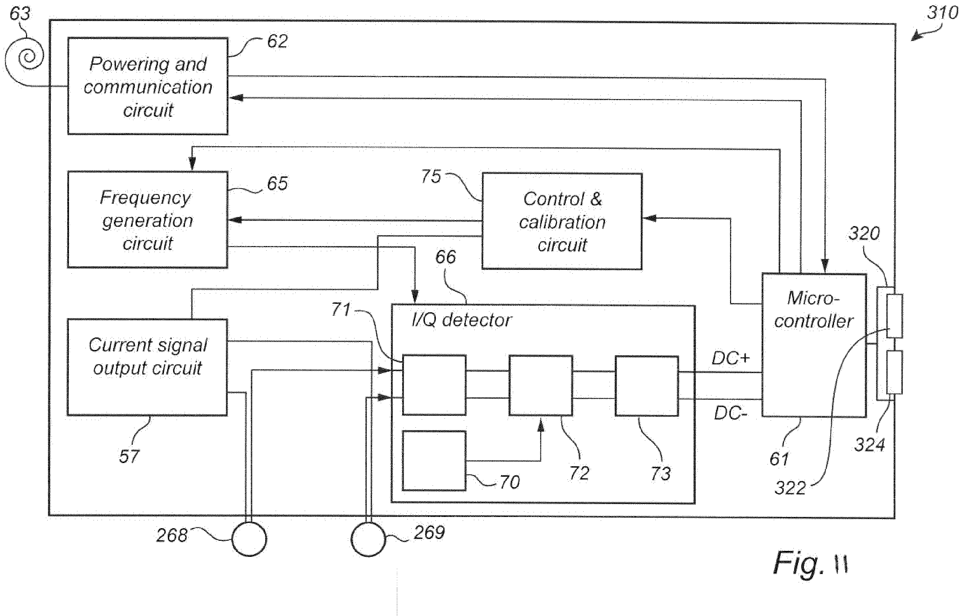

With reference to Fig. 11, another implantable impedance device or impedance sensor will be discussed in more detail. Fig. 11 shows a block diagram of this sensor. Like or similar parts or circuits shown in Fig. 8 are denoted with the same reference numeral in Fig. 11.

A powering and communication circuit 62 comprising analog circuits provides power to the sensor 310. The powering and communication circuit comprises a coil 63 for external powering by the reader module 14 using inductive coupling and the powering and communication circuit 62 is also configured to establish a communication mechanism with the reader module 14 using, for example, half duplex back-scattering serial technique. The powering and communication circuit 62 includes a full-wave rectifier circuit 64 which resonates with the coil 63, for example, at frequencies in a range between 10 - 15 MHz. The input to the powering and communication circuit 62 is an electromagnetic field produced by the coil 13 of the reading module 14. Output of the powering and communication circuit 62 is a DC voltage. The powering and communication circuit 62 is operatively connected to the microcontroller 61.

A frequency generation circuit 65 is configured to generate frequency reference clocks from signals having a frequency between 5 kHz to 50 MHz, and preferably in a range between 10 kHz to 20 MHz and more preferably in a range between 16 kHz to 16 MHz. These frequencies are used to generate sinusoidal current and I/Q waveforms for the I/Q impedance detection mechanism performed in an I/Q detector 66.

A current signal output circuit 67 is operatively connected to a pair of electrodes 268 and is configured to provide electrical current at predetermined frequencies to the electrodes 268. The electrodes 268 are configured to inject the electrical current into the body tissue, wherein the electrical current is passed from one of the electrodes 268 to the other of the electrodes 268 through the body. The current signal output circuit 67 is configured to provide the injected current at a plurality of frequencies in a range between1 kHz to 3 MHz, and preferably within a range between 1.5 kHz and 2.5 MHz, and more preferably in a range between 1.90 kHz and 2 MHz. The frequencies are 1.95 kHz, 3.9 kHz, 7.8125 kHz, 15.625 kHz, 31.25 kHz, 62.5 kHz, 125 kHz, 250 kHz, 500 kHz, 1MHZ and 2 MHz.

The resulting voltage caused by the current flowing between the pair of electrodes 268 and through the body tissue is detected at the electrodes 268. The electrodes 69 are also operatively connected to the detector 66, which receives the sensed voltage. The detector 66 comprises circuit for generating sinusoidal current waveform 70, amplifying circuits 71 for amplifying sensed voltage, multiplier 72 for multiplying the voltage with I/Q reference signals and low pass filter circuit 73 for low pass filtering the signals.

The detector 66 has one path to extract the I- and Q-components of the signal. The result of the I/Q demodulation is a DC signal, which entails that the extraction of the I and Q components can be performed when required. This is in contrast to prior art I/Q demodulation in communication systems, where phase and amplitude change over time and the processing therefore has to be performed in parallel.

A control and calibration circuit 75 is operatively connected to the microcontroller 61, current frequency generation circuit 65, the current signal output circuit 67 and the detector 66. The control and calibration circuit 75 is configured to control and/or calibrate the different circuits and to communicate with the microcontroller 61.

An optical detecting unit 320 including LED's 322 and a detector 324 is connected to the microcontroller 61. The LED's 322 may be two LED with different wavelengths to monitor oxygenated and deoxygenated blood that has different optical spectra and the detector may then be used to monitor oxygen saturation level. It should be noted that more than two LED's may be used.

During transportation of organs for implantation, ischemia is the major concern, which could be detected both by EIS and optical spectral analysis, however after implantation rejection and infection become of interest, and it is important to decide what problem is at hand since the counter measures are different. Thus, by adding LEDs and optical detector to the impedance sensor, it would become more accurate in differential diagnosis.

A number of other tissue conditions and analytes could be detected at the same time by choosing at least two LEDs of specific wavelengths, e.g. kreatinine which is a substance reflecting reduced kidney function. Other analytes reflect reduced liver function, and also general indicators such as temperature (thermistor), potassium level, sodium level, and pH could be included in the "button sized" sensor element and implanted for life.

In "A batteryless sensor ASIC for implantable Bio-impedance Applications", IEEE TBIOCAS, by S. Rodriquez et al., an example sensor, method and system are disclosed. A 2-kHz to 2-MHz bio-impedance sensor ASIC was designed and tested for implantable biomedical applications. The ASIC is designed in 150 nm CMOS technology and consumes 165 µA at 1.8 V when powered by an external reader. The proposed ASIC has been validated by performing electrical, electrochemical, and ex vivo measurements. All measurement results show that the proposed solution achieves around 1 Ωrms error when sensing a 100 Ω impedance (1% error). In real medical applications, the tissues present larger impedance values; therefore, making possible better sensitivity levels. The measurement results show that this ASIC is able to successfully meet the bio-impedance sensing requirements while at the same time allowing a miniature size, battery-less implantable solution. The bio-impedance ASIC was fabricated in a 150 nm 1.8 V CMOS process and bond-wired in a PLCC44 package for testing purposes. The circuit blocks occupy an active area of approximately 1.22 mm × 1.22 mm and consumes 165 µA.

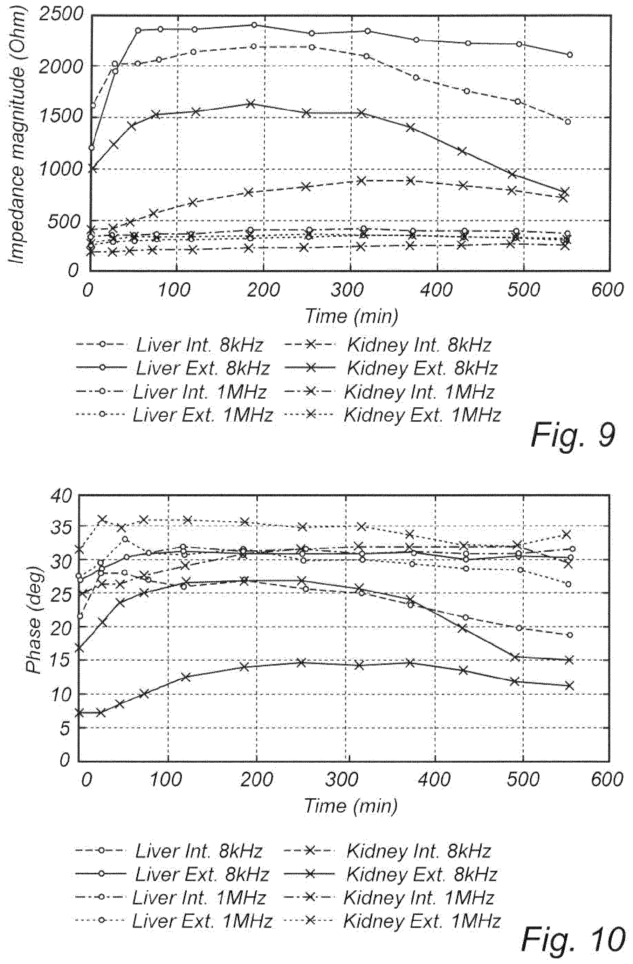

Ex vivo impedance measurements were performed on sheep's liver and kidney at 8 kHz and 1 MHz (1 point in the lower half of theβ dispertion and 1 point in the upper end of the β dispersion range of frequencies. The measurement procedure was as follows. The measurements started 25 minutes after circulation stopped (Time zero in Fig. 9 and Fig. 10), and lasted for several hours. A gold electrode probe was introduced in an incision done in each organ. In addition, another probe was fixed on the surface of the organs. The organs were deposited in plastic bags which were introduced in bowls filled with water. The water's temperature was constantly monitored and kept at around 37∘C. Fig. 9 and Fig. 10 show the measured impedance's magnitude and phase respectively for the probes introduced in the incisions (Int.) and the ones attached externally (Ext.). It is observed that the magnitude at low frequencies increases a few hundreds of Ω, remains relatively constant for some time, and then decreases in some cases below its initial value. On the other hand, the magnitude at 1 MHz remains relatively constant with values of a few hundreds of Ω. The measured phase at 8 kHz follows the pattern of the measured impedance at the same frequency: first it increases, peaks for some time, and then it decreases. This pattern can be partially explained by noticing that for
a very simple parallel RC model, an increase in R shifts the cut-off frequency to lower frequencies while increasing phase shift at higher frequencies. The behavior of the measured bioimpedances is in agreement with previous observations of ischemia in organs, where two factors inherent in R would be attributed to closing of gap junctions within a few hours after stop of circulation, followed later by rupture/lysis of cell membranes. A full decay of cell membranes would take another 10 hours or so, depending on temperature, and result in a lower impedance at the lower frequency than observed from the very beginning.
The ex vivo measurements confirm that the proposed ASIC accomplishes its target specifications, and therefore it can be successfully used to determine the bio-impedance of a variety of tissues in medical applications. The magnitude of the measured bio-impedances also confirm that the initial specifications, set for the minimum and maximum impedances, are at the correct levels. Furthermore, the measurements show that very accurate and stable measurements with errors in the order of 1 Ωrms are possible even in ex vivo conditions.

## Claims

1. A device (10) for measuring impedance in a subject, the device (10) being configured to be implanted within the body of the subject and being configured to measure impedance within a body tissue of the subject resulting from an electrical current flowing through the body tissue, wherein the body tissue is sub-dermal or subcutaneous tissue of the subject, comprising:
one pair of injection electrodes (68) configured for injection of electrical current into the body tissue, wherein the electrical current is passed from one of the injection electrodes to the other of the injection electrodes through the body;
one pair of sensing electrodes (69) configured to detect the resulting voltage caused by the current flowing between the pair of injection electrodes and through the body tissue;
a detector (66) operatively connected to the sensing electrodes (69) and configured to receive the voltage detected by the sensing electrodes (69), wherein the detector (66) is configured to measure the impedance of the body tissue based on the voltage detected by the pair of sensing electrodes (69);
a microcontroller (61) operatively connected to the detector and being configured to receive impedance signals from the detector and to provide control signals to the current signal output circuit;
a current signal output circuit (67) operatively connected to the microcontroller and the injection electrodes and being configured to provide electrical current at predetermined frequencies to the injection electrodes (68); and
a powering and communication circuit (62) including a coil configured to be powered by an electromagnetic field produced by an external coil the powering circuit being operatively connected to the microcontroller and configured to power the microcontroller, the current signal output circuit and the detector; wherein the microcontroller is programmed to determine a glucose level in the subject by correlating the measured impedance with a predetermined relationship between impedance and blood glucose levels, and
wherein the device for measuring impedance in a subject is a 2 kHz to 2-MHz bio-impedance sensor application-specific integrated circuit (ASIC), which is designed in 150nm CMOS technology and consumes 165µA at 1.8V when powered by an external reader.

2. The device according to claim 1, wherein the microcontroller (61) is configured to communicate the measured impedance to an external device (14) via the powering and communication circuit (62) and wherein the monitoring engine (25) is arranged in the external device.

3. The device according to claim 1, wherein the current signal output circuit (67) is configured to provide the injected current at a plurality of frequencies in a range between1kHz to 3 MHz, and preferably within a range between 1.5 kHz and 2.5 MHz, and more preferably in a range between 1.90 kHz and 2 MHz.

4. The device according to claim 1, further comprising a frequency generation circuit (65) operatively connected to the detector and being configured to generate reference signals having a frequency between 5 kHz to 50 MHz, and preferably in a range between 10 kHz to 20 MHz and more preferably in a range between 16 kHz to 16 MHz, and to deliver the reference signals to the detector (66).

5. The device according to claim 1, further comprising an I/Q demodulator (70, 71, 72, 73) wherein the I/Q demodulator (70, 71, 72, 73) comprises a multiplier configured to multiply the received voltage with the reference signal.

6. The device according to claim 1, wherein the detector (66) further comprises a voltage amplifier for amplifying the voltage sensed by the sensing electrodes.

7. The device according to claim 1, wherein the detector (66) further comprises a low pass filter for filtering the amplified signals.

8. The device according to claim 1, wherein the device (10) is configured to be implanted within the body of the subject sub-dermally or subcutaneously

9. The device according to claim 1, wherein the powering and communication circuit (62) is configured to communicate with an external communication device (14) using a back-scattering technique.

10. The device according to claim 1, further comprising an optical detecting unit including LED's and a detector, wherein the optical detecting unit is connected to the microcontroller (61).

## Patentansprüche

1. Vorrichtung (10) zur Impedanzmessung bei einem Individuum, wobei die Vorrichtung (10) dazu konfiguriert ist, in den Körper des Individuums implantiert zu werden, und dazu konfiguriert ist, Impedanz in einem Körpergewebe des Individuums zu messen, die aus einem durch das Körpergewebe fließenden elektrischen Strom resultiert, wobei das Körpergewebe subdermales oder subkutanes Gewebe des Individuums ist, umfassend:
ein Paar Einspeiseelektroden (68), die dazu konfiguriert sind, elektrischen Strom in das Körpergewebe einzuspeisen, wobei der elektrische Strom von einer der Einspeiseelektroden zu der anderen der Einspeiseelektroden durch den Körper geleitet wird; ein Paar Sensorelektroden (69), die dazu konfiguriert sind, die resultierende Spannung zu erfassen, die durch den Strom verursacht wird, der zwischen dem Paar von Einspeiseelektroden und durch das Körpergewebe fließt;
einen Detektor (66), der wirksam mit den Sensorelektroden (69) verbunden und dazu konfiguriert ist, um die von den Sensorelektroden (69) erfasste Spannung zu empfangen, wobei der Detektor (66) dazu konfiguriert ist, die Impedanz des Körpergewebes basierend auf der von dem Paar Sensorelektroden (69) erfassten Spannung zu messen;
einen Mikrocontroller (61), der wirksam mit dem Detektor verbunden und dazu konfiguriert ist, Impedanzsignale von dem Detektor zu empfangen und Steuersignale an der Stromsignal-Ausgangsschaltung bereitzustellen;
eine Stromsignal-Ausgangsschaltung (67), die wirksam mit dem Mikrocontroller und den Einspeiseelektroden verbunden und dazu konfiguriert ist, elektrischen Strom mit vorbestimmten Frequenzen an den Einspeiseelektroden (68) bereitzustellen; und
eine Leistungs- und Kommunikationsschaltung (62), die eine Spule beinhaltet, die dazu konfiguriert ist, durch ein von einer externen Spule erzeugtes elektromagnetisches Feld gespeist zu werden, wobei die Leistungsschaltung wirksam mit dem Mikrocontroller verbunden und dazu konfiguriert ist, den Mikrocontroller, die Stromsignal-Ausgangsschaltung und den Detektor zu speisen; wobei der Mikrocontroller dazu programmiert ist, durch Korrelieren der gemessenen Impedanz mit einer vorbestimmten Beziehung zwischen Impedanz und Blutglukosespielgen einen Glukosespiegel des Individuums zu bestimmen, und
wobei die Vorrichtung zur Impedanzmessung bei einem Individuum eine applikationsspezifische integrierte Schaltung (application-specific integrated circuit - ASIC) mit 2-kHz- bis 2-MHz-Bioimpedanzsensor ist, die in 150-nm-CMOS-Technologie ausgelegt ist und bei einer Speisung durch ein externes Lesegerät 165 µA bei 1,8 V verbraucht.

2. Vorrichtung nach Anspruch 1, wobei der Mikrocontroller (61) dazu konfiguriert ist, die gemessene Impedanz über die Leistungs- und Kommunikationsschaltung (62) an eine externe Vorrichtung (14) zu kommunizieren, und wobei die Überwachungsmaschine (25) in der externen Vorrichtung angeordnet ist.

3. Vorrichtung nach Anspruch 1, wobei die Stromsignal-Ausgangsschaltung (67) dazu konfiguriert ist, den eingespeisten Strom mit einer Vielzahl von Frequenzen in einem Bereich zwischen 1 kHz bis 3 MHz und vorzugsweise in einem Bereich zwischen 1,5 kHz und 2,5 MHz und mehr bevorzugt in einem Bereich zwischen 1,90 kHz und 2 MHz bereitzustellen.

4. Vorrichtung nach Anspruch 1, ferner umfassend eine Frequenzerzeugungsschaltung (65), die wirksam mit dem Detektor verbunden und konfiguriert ist, Referenzsignale zu erzeugen, die eine Frequenz zwischen 5 kHz bis 50 MHz und vorzugsweise in einem Bereich zwischen 10 kHz bis 20 MHz und mehr bevorzugt in einem Bereich zwischen 16 kHz bis 16 MHz aufweisen, und die Referenzsignale dem Detektor (66) zuzuführen.

5. Vorrichtung nach Anspruch 1, ferner umfassend einen I/Q-Demodulator (70, 71, 72, 73), wobei der I/Q-Demodulator (70, 71, 72, 73) einen Multiplizierer umfasst, der dazu konfiguriert ist, die empfangene Spannung mit dem Referenzsignal zu multiplizieren.

6. Vorrichtung nach Anspruch 1, wobei der Detektor (66) einen Spannungsverstärker zum Verstärken der von den Sensorelektroden registrierten Spannung umfasst.

7. Vorrichtung nach Anspruch 1, wobei der Detektor (66) ferner ein Tiefpassfilter zum Filtern der verstärkten Signale umfasst.

8. Vorrichtung nach Anspruch 1, wobei die Vorrichtung (10) dazu konfiguriert ist, subdermal oder subkutan in den Körper des Individuums implantiert zu werden.

9. Vorrichtung nach Anspruch 1, wobei die Leistungs- und Kommunikationsschaltung (62) dazu konfiguriert ist, unter Verwendung einer Rückstreutechnik mit einer externen Kommunikationsvorrichtung (14) zu kommunizieren.

10. Vorrichtung nach Anspruch 1, ferner umfassend eine optische Erfassungseinheit, die LED und einen Detektor beinhaltet, wobei die optische Erfassungseinheit mit dem Mikrocontroller (61) verbunden ist.

## Revendications

1. Dispositif (10) de mesure d'impédance chez un sujet, le dispositif (10) étant conçu pour être implanté dans le corps du sujet et étant conçu pour mesurer l'impédance dans un tissu corporel du sujet résultant d'un courant électrique circulant à travers le tissu corporel, dans lequel le tissu corporel est un tissu sous-dermique ou sous-cutané du sujet, comprenant :
une paire d'électrodes d'injection (68) conçue pour l'injection de courant électrique dans le tissu corporel, dans lequel le courant électrique passe de l'une des électrodes d'injection à l'autre des électrodes d'injection à travers le corps ;
une paire d'électrodes de détection (69) conçue pour détecter la tension résultante provoquée par le courant circulant entre la paire d'électrodes d'injection et à travers le tissu corporel ;
un détecteur (66) relié de manière fonctionnelle aux électrodes de détection (69) et conçu pour recevoir la tension détectée par les électrodes de détection (69), dans lequel le détecteur (66) est conçu pour mesurer l'impédance du tissu corporel sur la base de la tension détectée par la paire d'électrodes de détection (69) ;
un microcontrôleur (61) relié de manière fonctionnelle au détecteur et conçu pour recevoir des signaux d'impédance depuis le détecteur et pour fournir des signaux de commande au circuit de sortie de signal de courant ;
un circuit de sortie de signal de courant (67) relié de manière fonctionnelle au microcontrôleur et aux électrodes d'injection et conçu pour fournir un courant électrique aux électrodes d'injection (68) à des fréquences prédéterminées ; et
un circuit d'alimentation et de communication (62) comportant une bobine conçue pour être alimentée par un champ électromagnétique produit par une bobine externe, le circuit d'alimentation étant relié de manière fonctionnelle au microcontrôleur et conçu pour alimenter le microcontrôleur, le circuit de sortie de signal de courant et le détecteur ; dans lequel le microcontrôleur est programmé pour déterminer un taux de glucose chez le sujet en corrélant l'impédance mesurée avec une relation prédéterminée entre l'impédance et les taux de glucose dans le sang, et
dans lequel le dispositif de mesure d'impédance chez un sujet est un circuit intégré à application spécifique (ASIC) de capteur de bioimpédance de 2 kHz à 2 MHz, qui est conçu selon une technologie CMOS de 150 nm et consomme 165 µA à 1,8 V lorsqu'il est alimenté par un lecteur externe.

2. Dispositif selon la revendication 1, dans lequel le microcontrôleur (61) est conçu pour communiquer l'impédance mesurée à un dispositif externe (14) par l'intermédiaire du circuit d'alimentation et de communication (62) et dans lequel le moteur de surveillance (25) est agencé dans le dispositif externe.

3. Dispositif selon la revendication 1, dans lequel le circuit de sortie de signal de courant (67) est conçu pour fournir le courant injecté à une pluralité de fréquences dans une plage comprise entre 1 kHz et 3 MHz, et de préférence dans une plage comprise entre 1,5 kHz et 2,5 MHz, et de manière davantage préférée dans une plage comprise entre 1,90 kHz et 2 MHz.

4. Dispositif selon la revendication 1, comprenant en outre un circuit de génération de fréquence (65) relié de manière fonctionnelle au détecteur et conçu pour générer des signaux de référence présentant une fréquence dans une plage comprise entre 5 kHz et 50 MHz, et de préférence dans une plage comprise entre 10 kHz et 20 MHz, et de manière davantage préférée dans une plage comprise entre 16 kHz et 16 MHz, et pour délivrer les signaux de référence au détecteur (66).

5. Dispositif selon la revendication 1, comprenant en outre un démodulateur I/Q (70, 71, 72, 73) dans lequel le démodulateur I/Q (70, 71, 72, 73) comprend un multiplicateur conçu pour multiplier la tension reçue par le signal de référence.

6. Dispositif selon la revendication 1, dans lequel le détecteur (66) comprend en outre un amplificateur de tension pour amplifier la tension captée par les électrodes de détection.

7. Dispositif selon la revendication 1, dans lequel le détecteur (66) comporte en outre un filtre passe-bas pour filtrer les signaux amplifiés.

8. Dispositif selon la revendication 1, dans lequel le dispositif (10) est conçu pour être implanté dans le corps du sujet de manière sous-dermique ou sous-cutanée.

9. Dispositif selon la revendication 1, dans lequel le circuit d'alimentation et de communication (62) est conçu pour communiquer avec un dispositif de communication externe (14) à l'aide d'une technique de rétrodiffusion.

10. Dispositif selon la revendication 1, comprenant en outre une unité de détection optique comportant des LED et un détecteur, dans lequel l'unité de détection optique est reliée au microcontrôleur (61).
